(19) Europäisches Patentamt
European Patent Office
Office européen des brevets

(11) EP 4 671 355 A1

(12) EUROPEAN PATENT APPLICATION
published in accordance with Art. 153(4) EPC

(43) Date of publication:
31.12.2025 Bulletin 2026/01

(51) International Patent Classification (IPC):
C12M 3/00 (2006.01)          C12N 5/073 (2010.01)
C12M 1/34 (2006.01)

(21) Application number: 23924028.6

(52) Cooperative Patent Classification (CPC):
C12M 1/34; C12M 3/00; C12N 5/06

(22) Date of filing: 22.02.2023

(86) International application number:
PCT/JP2023/006407

(87) International publication number:
WO 2024/176374 (29.08.2024 Gazette 2024/35)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Asada Ladies Clinic
Nagoya-shi, Aichi, 450-0002 (JP)

(72) Inventors:
• ASADA Yoshimasa
  Nagoya-shi, Aichi 450-0002 (JP)
• FUKUNAGA Noritaka
  Nagoya-shi, Aichi 450-0002 (JP)

(74) Representative: Kuhnen & Wacker
Patent- und Rechtsanwaltsbüro PartG mbB
Prinz-Ludwig-Straße 40A
85354 Freising (DE)

(54) **DISH FOR EMBRYO CULTURE AND METHOD FOR COLLECTING EMBRYO CULTURE SOLUTION FOR CHROMOSOME ANALYSIS BY USING SAID DISH**

(57) In an embryo culture dish that includes a plurality of wells that each contain an embryo together with a culture solution, the plurality of wells are disposed independently and adjacently at a bottom part of the embryo culture dish, a recess part that can retain the culture solution together with the well is formed per well at the bottom part independently from another recess part, a movement block part that permits circulation of the culture solution and blocks movement of the embryo is provided between the well and the recess part, and a volume of the recess part is equal to or more than a volume of a culture solution to be sucked up for testing for aneuploidy of a chromosome of the embryo cultured in the well. By so doing, it is possible to suck up the culture solution for testing for aneuploidy of chromosomes of an embryo to be cultured, and dispose the wells close to each other that contain a plurality of embryos.

Fig.5

**Description**

FIELD

The present disclosure relates to a dish used for embryo culture, and a method for sampling a chromosome analysis embryo culture solution using this dish.

RELATED ART

A social demand for infertility treatment has been increasing, and a technique of culturing treated eggs and embryos (hereinafter, collectively referred to as embryos) subjected to fertilization treatment, and various methods such as testing accompanying this technique have been variously proposed. Such a method is described in JP 2014-507664 W or the like. In recent years in particular, needs for chromosome analysis of an embryo that is being cultured are increasing, and there is also proposed a method for analyzing DNAs freed from an embryo that exists in a culture solution in which the embryo is cultured, and analyzing chromosome aneuploidy of the embryo and the like. Such testing requires a predetermined amount of a culture solution. In a current situation, approximately several $\mu$l to several tens of $\mu$l of a culture solution is sucked up, and used for testing.

However, in recent years, the number of embryos to be cultured at a time by an embryo culture device is increased, the embryo is contained in each of a plurality of microwells, that is, for example, 25 microwells (5 x 5) provided to one dish and are cultured, and a necessary amount of a culture solution for testing cannot be sucked up from these microwells. The diameter of an egg of a person is approximately 150 to 200 $\mu$m, and a little culture solution can only be sucked up from, for example, a microwell whose diameter is approximately 300 $\mu$m. What is not preferable is that, if a necessary amount of a culture solution is sucked up in this state, an embryo culture environment may greatly change.

Furthermore, even if the volume of a well that contains an embryo is simply increased, a distance between adjacent wells increases, and various problems may occur. For example, it is concerned that, in an embryo culture device on which a time-lapse function of photographing an embryo that is being cultured is mounted, the number of wells that can be imaged at a time decreases, and the structure of a photographing device becomes complicated. A distance for transferring an embryo between wells increases, and, for example, a probability that a mistake is induced at a time of transfer also increases.

SUMMARY

The present disclosure can be implemented as following aspects or application examples. One aspect of the present disclosure is an aspect of an embryo culture dish that includes a plurality of wells that each contain an embryo together with a culture solution. In this embryo culture dish, the plurality of wells may be disposed independently and adjacently at a bottom part of the embryo culture dish, a recess part that can retain the culture solution together with the well may be formed per well at the bottom part independently from another recess part, a movement block part that permits circulation of the culture solution and blocks movement of the embryo may be provided between the well and the recess part, and a volume of the recess part may be equal to or more than a volume of a culture solution to be sucked up for testing for aneuploidy of a chromosome of the embryo cultured in the well.

According to such an embryo culture dish, the plurality of wells are adjacent, so that, by using the embryo culture dish for an embryo culture device, distances between the plurality of wells are short and are easy to handle, and it is possible to perform processing of sucking up the culture solution for testing for aneuploidy of chromosomes of an embryo while suppressing an influence on the embryo that is being cultured. The distances between the plurality of wells are short, so that it is easy to move an embryo in a well, and it is possible to reduce mistakes at a time of transfer. Furthermore, by using the embryo culture dish for a time-lapse embryo culture device, it is possible to image a plurality of wells at a time. Consequently, it is easy to perform non-invasive testing for aneuploidy of chromosomes on embryos, and it is possible to expect that an embryo transfer success rate (a pregnancy rate of a patient) in consideration of results of testing for aneuploidy increases.

BRIEF DESCRIPTION OF DRAWINGS

FIG. 1 is a plan view of an embryo culture device according to a first embodiment;
FIG. 2 is a plan view illustrating an embryo culture dish disposed in a culture chamber;
FIG. 3 is a III-III arrow view in FIG. 2;
FIG. 4 is an explanatory view illustrating an electrical configuration of the embryo culture device;
FIG. 5 is a plan view illustrating an example of arrangement of a first container part and a second container part of the embryo culture dish;
FIG. 6 is a VI-VI arrow view in FIG. 5;
FIG. 7 is an explanatory view illustrating a state where a culture solution is filled in the first container part and the second container part;
FIG. 8 is an explanatory view illustrating a state where the culture solution in the first container part and the second container part is sucked out;
FIG. 9 is a flowchart illustrating contents of sample/testing processing of a chromosome analysis embryo culture solution;
FIG. 10 is an explanatory view illustrating an example of a display screen of a chromosome aneuploidy detector.

FIG. 11 is a plan view illustrating the shape of the embryo culture dish to a second embodiment;

FIG. 12 is a plan view illustrating a modified example of an embryo culture dish according to the second embodiment;

FIG. 13 is an explanatory view illustrating the first container part and the second container part according to another embodiment;

FIG. 14 is a flowchart illustrating an outline of embryo culture/storage/transplantation treatment; and

FIG. 15 is an explanatory view illustrating a state where the embryo culture dish is held by a hand.

DETAILED DESCRIPTION

A. First Embodiment:

(A-1) Hardware Configuration:

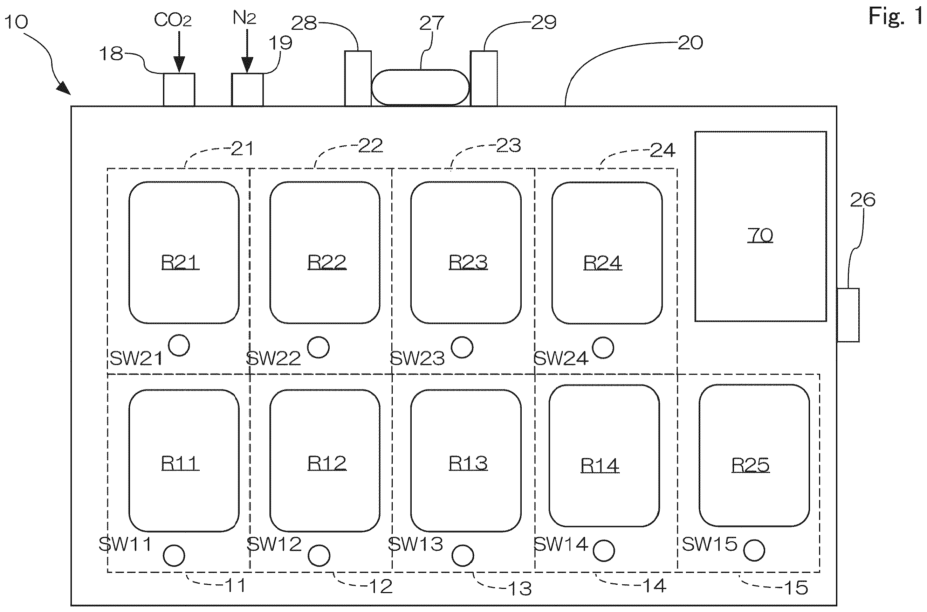

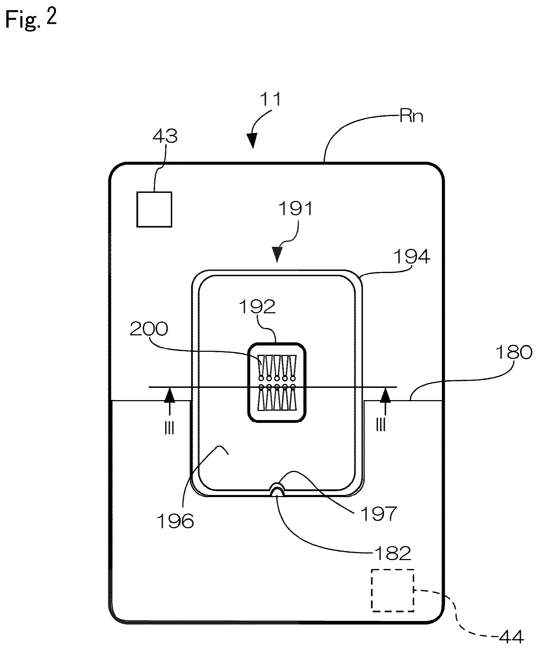

FIG. 1 is a plan view of an embryo culture device (also referred to as an incubator) 10, and FIG. 2 is an explanatory view schematically illustrating an embryo culture dish disposed in one culture chamber of the embryo culture device 10. This embryo culture device 10 cultures an egg (hereinafter, referred to as a treated egg) subjected to in vitro fertilization treatment for a certain period under a predetermined culture environment, that is, at a constant temperature and a constant humidity. The treated egg is not necessarily a fertilized egg, and therefore is cultured for the predetermined period in the embryo culture device 10. Note that there may be a case of intra-cytoplasmic sperm injection where in vitro fertilization treatment is performed under a microscope, or there may be also cases of normal in vitro fertilization where eggs and sperms are contained together in a predetermined container. A method for fertilizing a treated egg to culture does not matter.

As illustrated in FIG. 1, this embryo culture device 10 includes nine culture units in total that perform embryo culture, and includes five culture units 11 to 15 aligned at a lower stage and four culture units 21 to 24 aligned at an upper stage. Hereinafter, in a case where members related to the culture units will be collectively described, the members will be described as the culture units 11 to 24. Naturally, the number of culture units does not matter. The culture units 11 to 24 include culture chambers R11 to R24, lid parts 17 that seal the culture chambers R11 to R24, and switches SW11 to SW24, respectively. By operating the switches SW11 to SW24, the lid parts 17 of the corresponding culture units 11 to 24 are opened and closed by an unillustrated driving mechanism. Naturally, the culture units 11 to 24 may have structures that are manually opened and closed. The culture chambers R11 to R24 sides of the lid parts 17 are provided with seal parts of silicone rubber, and, when the lid parts 17 are closed, the interiors of the culture chambers R11 to R24 are kept in an airtight state by these seal parts.

Carbon dioxide gas ($CO_2$) and nitrogen gas ($N_2$) are supplied to the embryo culture device 10 from an external gas cylinder. Gas ports 18 and 19 to which these gases are supplied are provided on the back surface of the embryo culture device 10. Furthermore, filter ports 28 and 29 to which a filter 27 is attached are also provided to the back surface of the embryo culture device 10. This filter 27 removes foreign materials such as dust in outdoor air taken in by an unillustrated built-in pump. The carbon dioxide gas $CO_2$ and the nitrogen gas $N_2$ taken in from the gas ports 18 and 19 and air taken in through the filter 27 are mixed at a predetermined rate in the embryo culture device 10 to generate a mixture. A rate of each gas in the mixture is measured by an unillustrated sensor, and is kept at the same rate at all times.

A case main body 20 has the surface that is provided with a display 70. The surface of the display 70 is a touch panel, and can display various information by tapping a displayed button or the like. Furthermore, the case main body 20 is provided with a general-purpose connector 26 such as a USB-C or a thunderbolt (registered trademark), and various information can be also input therein by connecting an input device such as keyboard or a pointing device such as a mouse thereto. A display example of the display 70 and a case where another device is connected to the general-purpose connector 26 will be described later.

FIG. 2 illustrates a state where the lid part 17 of the culture unit 11 is opened. The culture chambers R11 to R24 are prepared for the culture units 11 to 24. Since the structures of the culture chambers R11 to R24 are the same, the culture chamber R11 of the culture unit 11 will be cited as an example and described below. The culture chamber R11 is provided with a holding frame 180 at which a dish 191 is placed. A bottom surface of each of the culture chambers R11 to R24 is provided with a supply port 43 and an exhaust port 44. The supply port 43 is an opening for supplying the mixture to the culture chamber R11. Furthermore, the exhaust port 44 is an opening for refluxing the mixture. The exhaust port 44 is provided at a position covered by the holding frame 180. As already described above, since the culture chamber R11 is substantially airtight when the lid part 17 is closed, an internal gas environment is kept constantly. However, the mixture is supplied from the supply port 43 and is exhausted from the exhaust port 44 to make a temperature distribution in the culture chamber R11 uniform.

The culture chamber R11 has the sidewall and the bottom surface that are provided with an unillustrated panel heater. The panel heater keeps the inside of the culture chamber R11 at a constant temperature. Note that, although description will be omitted, an unillustrated temperature sensor and a gas concentration sensor, and, moreover, a humidity sensor and the like are provided to each of the culture chambers R11 to R24 in addition to the heater, and can detect a temperature, a gas concentration, and a humidity in the culture chamber R11. Signals from these sensors are fed back to keep the constant temperature and the constant humidity in the culture chamber R11 as a result, and constantly keep the gas concentration. Note that, by constantly keeping a temperature, a humidity, a gas concentration, and the like of the mixture supplied from the supply port 43 instead of detecting the temperature or the like in the culture chamber R11, the environment in the culture chamber R11 may be kept constantly.

The dish 191 that contains an embryo is disposed on the holding frame 180 of the culture chamber R11. The dish 191 illustrated in FIG. 2 has a substantially rectangular shape surrounded by an outer circumferential wall 194, and includes at a center part of a bottom part 196 two rows of five container parts 200, that is, the 10 container parts 200 in total that are surrounded by a wall 192 and contain embryos. A detailed configuration of the container part 200 will be described later. One portion of the outer circumferential wall 194 of the dish 191 is provided with a recess portion 197, and, by aligning this recess portion 197 to a protrusion part 182 provided to the holding frame 180 and setting the dish 191 to the holding frame 180, it is possible to dispose the dish 191 at a correct position. Here, the correct position means an appropriate position for imaging a microwell 195 described later and provided to the container part 200.

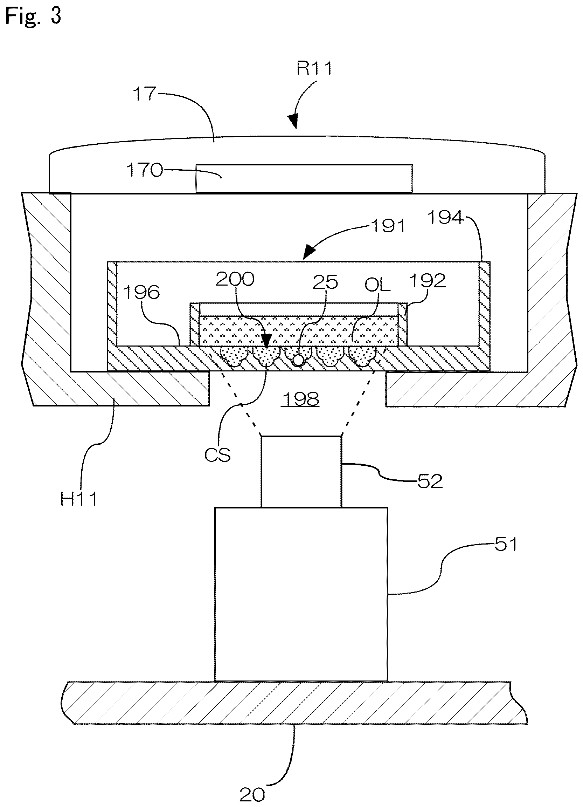

A configuration of imaging the dish 191 from the bottom part 196 side will be described with reference to FIG. 3 that is a III-III arrow view of FIG. 2. In FIG. 3, reference numeral H11 denotes a continuous sidewall surface and bottom part that form the culture chamber R11. As illustrated in FIG. 3, an illumination unit 170 provided inside the lid part 17 is disposed just above the dish 191. Furthermore, the bottom part H11 of the culture chamber R11 is provided with an opening 198 just below a position meeting the center part of the dish 191, and a camera module 51 and a lens module 52 attached to the case main body 20 are disposed therein. The dish 191 is illuminated by the illumination unit 170 provided to the lid part 17, and an embryo 25 contained in the container part 200 is imaged by the camera module 51. Note that, as illustrated in FIG. 3, the container part 200 is filled with a culture solution CS, and the embryo 25 is immersed in this culture solution CS. Furthermore, a mineral oil OL is flown in a region surrounded by the wall 192 in this state to prevent evaporation of the culture solution CS, and covers the container part 200.

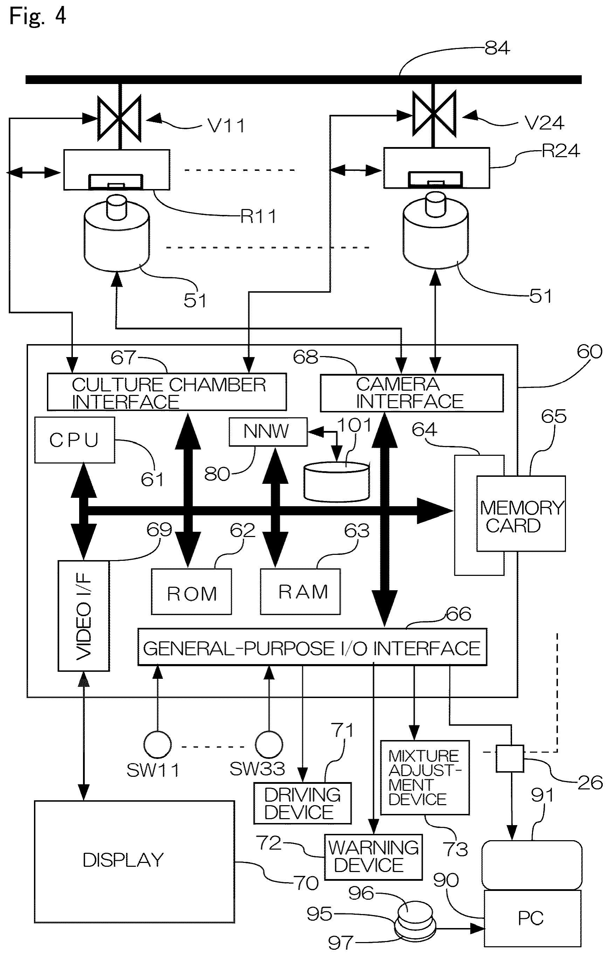

Next, processing performed in the embryo culture device 10 will be described. FIG. 4 is an explanatory view illustrating an electrical configuration of the embryo culture device 10. As illustrated in FIG. 4, the embryo culture device 10 is provided with a control unit 60. The control unit 60 includes a known CPU 61, ROM 62, and RAM 63, and, in addition, a memory interface 64 for exchanging data with a memory card 65, a general-purpose I/O interface 66 that exchanges a signal with an external device, a culture chamber interface 67 that exchanges a signal for controlling environments in the culture chambers R11 to R24, a camera interface 68 that instructs imaging or acquires captured images from the camera module 51, a video interface (abbreviated as a video I/F) 69 that displays images on the display 70, and the like.

For the CPU 61, a high-speed processor has been used that has, for example, a built-in function that is a DSP that processes fertilization determination or the like based on an image to be described later at a high speed, and vector computation function for performing determination processing of a neural network or the like. The ROM 62 stores a program that implements processing including time-lapse control processing to be described later in the embryo culture device 10. The CPU 61 implements necessary processing and control by appropriately reading such a program from the ROM 62, and expanding and executing the program in the RAM 63. In the memory card 65, images captured by the camera module 51 and, in addition, information related to culture environment such as the temperature in each of the culture chambers R11 to R24 is recorded. Instead of the memory card 65, a magnetic storage medium such as a hard disk, a semiconductor storage medium such as an SSD, or the like may be used. Furthermore, information may be stored in a so-called cloud connected via a network.

The display 70 is provided with the touch panel on the surface, and buttons or the like displayed on the display 70 can be selected by touching the display 70. The display 70 is provided integrally with the embryo culture device 10 in the present embodiment, yet may be provided separately from the embryo culture device 10 and connected with the embryo culture device 10 by wire or wirelessly. Alternatively, the embryo culture device 10 and a computer may be connected via a network or the like, and a display of the computer may be used as the display 70. Alternatively, a terminal such as a mobile telephone or a tablet that is highly portable may be used as the display 70.

The general-purpose I/O interface 66 is connected to the switches SW11 to SW24 provided to the embryo culture device 10, a driving device 71 that opens or closes the lid parts 17, a warning device 72 that sets out a warning sound, a mixture adjustment device 73 that adjusts the mixture, and the like. The mixture adjustment device 73 includes a pressure regulation valve that adjusts pressures of carbon dioxide gas, nitrogen gas, and the like to be supplied to the gas ports 18 and 19, a pump that takes in the atmosphere through the filter 27, a pump that feeds the mixture to the culture chambers R11 to R24, and the like. The culture chamber interface 67 is connected with the panel heaters provided to the culture chambers R11 to R24, control valves V11 to V24 that control a supply amount of the mixture, and the like. The control valves V11 to V24 are provided to pipes that go to the culture chambers R11 to R24 from a mixture supply pipe 84 that supplies the mixture.

The general-purpose I/O interface 66 is connected with a computer (hereinafter, abbreviated as a PC) 90 via the general-purpose connector 26. The PC 90 includes a rotary control device 95 in addition to a display 91, an unillustrated keyboard, and the like. The control device 95 includes on a base part 97 of a substantially circular shape a dial 96 that has a smaller diameter than that of the base part 97, and, by turning the dial 96 to the left and the right, the dial 96 can be used to move multiple time-lapse images displayed on the display 91 chronologically back and forth, and display the time-lapse images. Such a device will be also referred to as a rotary selector. The dial 96 not only rotates to the left and the right, but also can be pushed, and implements an operation of "determination" similarly to an operation of a mouse button when selecting one currently displayed image. Naturally, the base part 97 may be provided with dedicated buttons, and determination and selection operations may be assigned thereto. The number of time-lapse images are $6 \times 24 = 144$ per embryo if the embryo is imaged every 10 minutes for 24 hours. When, for example, determination on fertilization is checked by quickly referring to these multiple images, such a rotary pointing device is useful.

(A-2) Details of Dish:

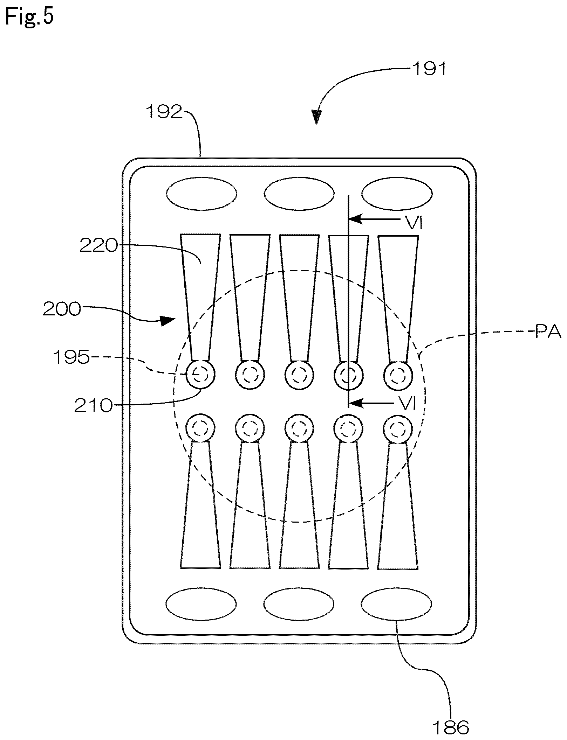

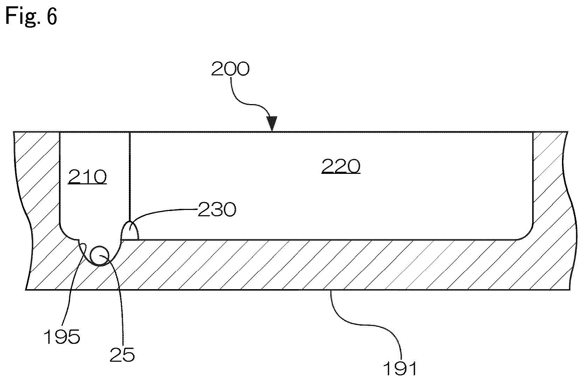

FIG. 5 is an enlarged view of a portion of the dish 191 surrounded by the wall 192, and FIG. 6 is a VI-VI cross-sectional view in FIG. 5. As illustrated in FIG. 5, the two rows of the five container parts 200 are vertically and symmetrically aligned in FIG. 5 in a range of a substantially rectangular shape surrounded by the wall 192. A plurality of drop arrangement parts 186 at which the culture solution CS for cleaning are provided on the outer side of the 10 container parts 200 and near the wall 192. The drop arrangement part 186 may allow a drop of the culture solution CS to be simply put on the bottom part 196. However, the bottom part 196 may be provided with a recess or provided with a hydrophilic region to make it easy to put the culture solution CS.

Each container part 200 is formed in a shape formed by connecting a first container part 210 of a substantially circular shape and a second container part 220 of a trapezoidal shape that is long in an up-down direction in FIG. 5. This first container part 210 corresponds to a well, and the second container part 220 corresponds to a recess part. At the center of the first container part 210/, the microwell 195 is further formed. The microwell 195 has a larger diameter than that of the embryo 25. Hence, the 10 microwells 195 are disposed close to each other, and settle in one imaging area PA of the camera module 51 disposed below the dish 191. That is, it is possible to photograph regions in which all the microwells 195 exist by performing imaging once, and, if the embryos 25 are contained in the microwells 195, the states of the embryos 25 can be imaged at a time. The photographed images are divided into images of the respective embryos 25, and stored in the memory card 65 or the like.

The first container part 210 and the second container part 220 are connected via a connection portion as illustrated in FIG. 6. This portion is a passage portion. At this passage portion, partition parts 230 that are slightly higher than the bottom surface of the second container part 220 are formed as a movement block part. These partition parts 230 are provided protruding from both sides in a width direction of a connected part toward the center part, and the partition parts 230 protruding from the both sides face each other with a slight gap left at the center of the connected part, that is, a position at which a VI-VI cut line passes. This gap is smaller than an assumed minimum diameter of the embryo 25, and is approximately 50 $\mu$m in the present embodiment. Hence, the embryo 25 contained in the microwell 195 does not move toward the second container part 220 side due to the motion of the culture solution CS or the like even when each container part 200 is filled with the culture solution CS.

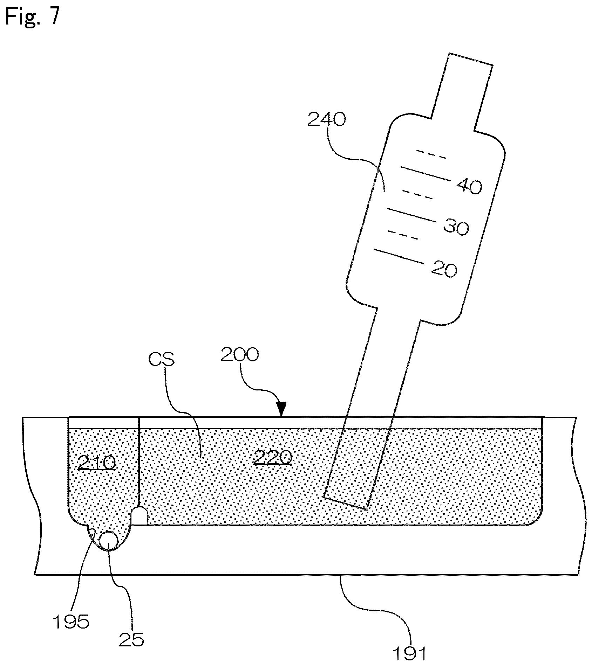

Filling and suction of the culture solution CS to and from the container part 200 will be described. FIG. 7 illustrates that the culture solution CS is filled into the container part 200 using a pipette 240. The culture solution CS is filled up to an upper limit of the container part 200. Since the volume of the container part 200 is determined in advance, and the volume of the container part 200 is approximately 50 $\mu$l in relation to 30 $\mu$l determined as the volume of the culture solution to be suctioned and described later in the present embodiment, 40 $\mu$l of the culture solution CS may be sucked by the pipette 240 and transferred to the container part 200. Naturally, if the volume of the culture solution to be suctioned becomes further smaller, the volume of the container part 200 may be set according to the volume of the culture solution. Then, the culture solution CS is covered with the mineral oil OL, a glass capillary is disposed above the microwell 195, and the embryo 25 is disposed inside the microwell 195.

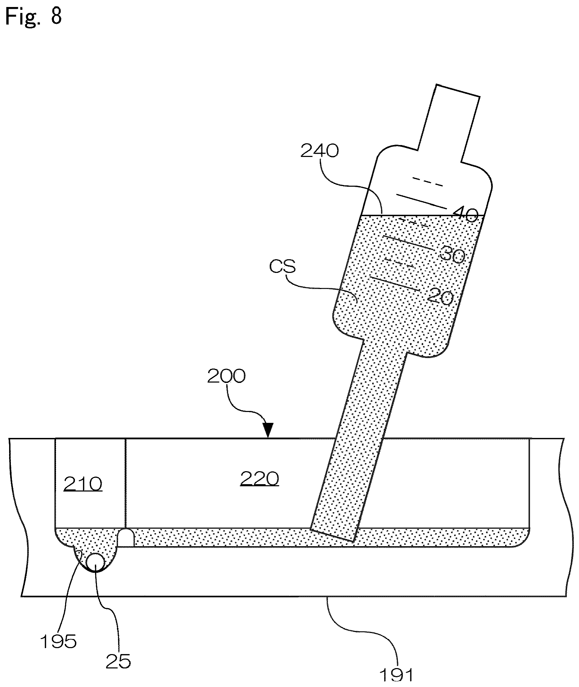

In the present embodiment, after the embryo 25 continues to be cultured, the culture solution CS of the container part 200 is suctioned, and detection for aneuploidy of chromosomes is performed. In this case, as illustrated in FIG. 8, the culture solution CS is sucked up from the container part 200 using the pipette 240. Although the culture solution CS still remains at the bottom of the second container part 220 in FIG. 8, the first container part 210 and the second container part 220 continue to each other via the gap between the partition parts 230. Therefore, if all of the culture solution CS of the second container part 220 is sucked up, the water level of the culture solution CS in the first container part 210 matches with the bottom part of the second container part 220. Even in this state, the embryo 25 is immersed in the culture solution CS. Furthermore, even when the culture solution CS in the first container part 210 moves toward the second container part 220 as the culture solution CS is sucked up by the pipette 240, the gap between the partition parts 230 is sufficiently narrow compared to the diameter of the embryo 25, so that the embryo 25 does not flow out from the microwell 195 to the second container part 220 due to the flow of the culture solution CS.

(A-3) Embryo Culture/Inspection:

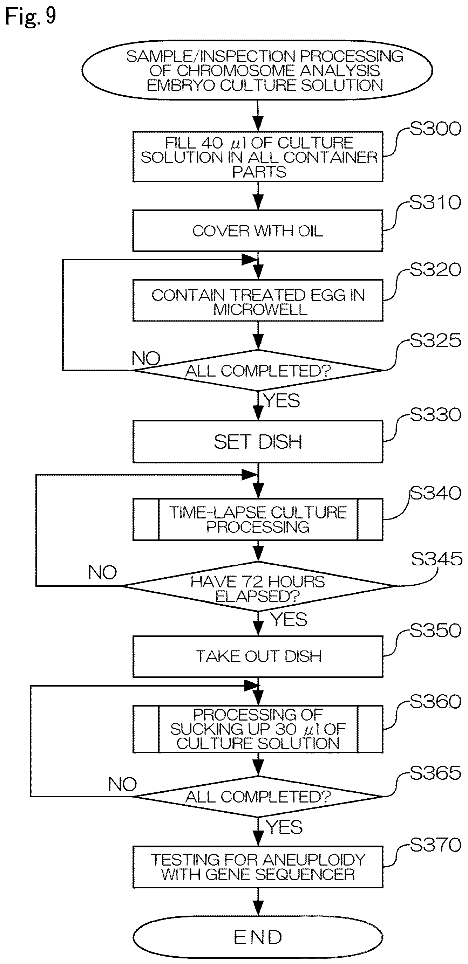

Next, testing of an embryo to be cultured using this dish 191 will be described. FIG. 9 is a flowchart illustrating a sample/testing processing routine of a chromosome analysis embryo culture solution. Although a treated egg subjected to fertilization treatment is cultured by the embryo culture device 10 in the present embodiment, testing for aneuploidy of chromosomes of an embryo that is being cultured is performed during culturing. This testing is called Preimplantation Genetic Testing for Aneuploidy (PGT-A). The preimplantation genetic testing for aneuploidy is usually performed by sampling approximately six to ten cells from an embryo biopsy of a blastocyst that is being cultured, that is, a trophectoderm of the blastocyst, a culture solution in which an embryo has been immersed is used in the present embodiment. DNAs freed from the embryo that is being cultured are exist in the culture solution, and testing for aneuploidy of chromosomes can be performed by putting these DNAs in a gene sequencer.

When this processing is started, 40 $\mu$l of a culture solution is filled in each one of the 10 container parts 200 provided to the dish 191 first (step S300). For the culture solution CS, a single medium (one-step type culture solution) doped with phenol red is used in the present embodiment. Since the volume of the container part 200 is 50 $\mu$l, even if the 40 $\mu$l of the culture solution CS is filled in the container part 200, the culture solution CS does not spill over from the container part 200. After the culture solution CS is filled in all of the 10 container parts 200, the mineral oil OL is filled on the inner side of the wall 192, and each container part 200 filled with the culture solution CS is covered with the mineral oil OL (step S310).

In this state, the embryo 25 is next contained in the microwell 195 of each container part 200 (step S320). At this time, if necessary, treatment of removing bubbles existing in the culture solution CS is performed. The one embryo 25 is contained in the one microwell 195, and this processing is repeated until containing the embryos 25 in all of the microwells 195 is completed (step S325).

After the embryos 25 are contained in all of the microwells 195, this dish 191 is next set to one of the culture chambers R11 to R24 of the culture units 11 to 24 of the embryo culture device 10 (step S330). More specifically, the lid part 17 of any one of the culture chambers is opened, and the dish 191 is adjusted to the holding frame 180 and disposed. Then, the lid part 17 is closed to start culturing.

As already described above, the embryo culture device 10 keeps the environment in the culture chamber R11 in a state suitable for culturing, and performs time-lapse culture processing of culturing the embryo (step S340) while imaging the embryo 25 contained in the microwell 195 of each container part 200 of the dish 191 at a predetermined interval using the camera module 51 that photographs the dish 191 from below. This time-lapse culture processing is continued until 72 hours determined as a predetermined time have elapsed from start of culturing (step S345).

When 72 hours have elapsed from start of culturing, the lid part 17 is opened and the dish 191 is taken out from the culture chamber R11 (step S350), and processing of sucking up the culture solution CS from each container part 200 is performed (step S360). More specifically, as illustrated in FIG. 8, the distal end of the pipette 240 is inserted in the second container part 220 of each container part 200 to suck up the culture solution CS. At this time, all of the culture solution CS in the second container part 220 is sucked up. If all of the culture solution CS in the second container part 220 is sucked up, the culture solution CS in the first container part 210 continuing to this second container part 220 is also sucked up. However, the microwell 195 at the bottom part of the first container part 210 is provided at the position deeper than the bottom part of the second container part 220, so that the culture solution CS in the microwell 195 is not sucked. Consequently, even when the pipette 240 is inserted in the second container part 220 and all of the culture solution CS in the second container part 220 is sucked, the embryo 25 is still kept in an immersed state in the culture solution CS. Note that, when the culture solution CS is sucked by the pipette 240, the culture solution CS may be sucked while measuring the culture solution CS such that the quantity of the culture solution CS is 30 $\mu$l. The sucked culture solution CS is individually put in an analysis sample case of the gene sequencer to be described later.

The embryo 25 remaining in the microwell 195 may continue to be further cultured by filling the new culture solution CS from the second container part 220, or may be taken out from the microwell 195 once and frozen and stored in preparation for transplantation. Although the culture solution CS is sucked up 72 hours after start of culturing in the above example, by taking an advantage of that the embryo culture device 10 is the time-lapse embryo culture device, an embryo may continue to be observed, standby may be performed until completion of a blastocyst, and the culture solution CS may be sucked up. In this case, the culture solution CS is usually sucked up approximately 120 hours after start of culturing. Note that completion of the blastocyst may be determined by an AI that has performed supervised machine learning on photographed images in advance.

Such processing is repeated for all of the container parts 200 of the one dish 191 and, when the culture solution CS is sucked from all of the container parts 200 and is transferred to the analysis sample case (step S365), the analysis sample case is set to the gene sequencer, and the gene sequencer performs testing for aneuploidy (step S370). In the present embodiment, "MiSeq" made by Illumina, Inc has been used as the gene sequencer. Thus, this processing routine proceeds to "END" and is ended.

The culture solution CS contained in the analysis sample case includes fragments of DNAs freed from an embryo that is being cultured. The fragments of the DNAs are originally separated from 23 pairs of genes, and include information on original genes. Hence, by analyzing a culture solution contained in the analysis sample case by a sequencer (so-called next-generation sequencer) that can use the NGS method for setting to a semiconductor chip a template prepared using normal gene samples of a human, it is possible to easily analyze deficiency and excess of the 23 pairs of genes. As such a next-generation sequencer, for example, "NextSeq550" made by liiumina, Inc, "Ion GneStudio GX5" made by Ion Torrent, or the like can be also used.

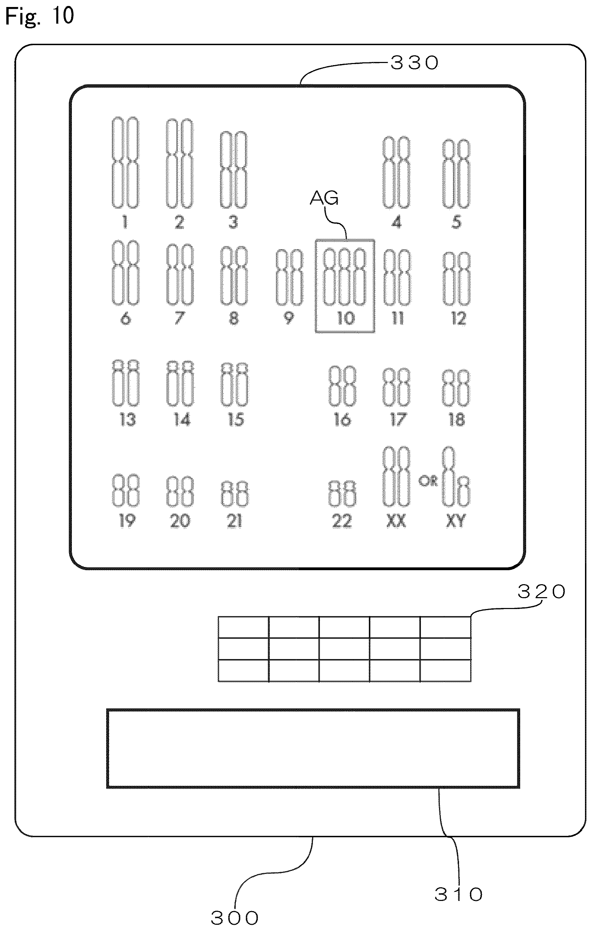

FIG. 10 illustrates an example of an analysis result. As illustrated in FIG. 10, a next-generation sequencer 300 that is an analyzer is provided with a container part 310 that contains the analysis sample case, an operation unit 320 that instructs work such as template preparation or start of analysis, a display unit 330 that displays an analysis result, and the like. When the analysis sample case to which the culture solution CS that has been culturing each embryo 25 has been transferred is set to the container part 310 of the next-generation sequencer 300 for which preparation such as template preparation has been finished, and start of analysis is instructed, the number of genes included in the culture solution CS is displayed as illustrated in FIG. 10 after several hours to several tens of hours.

In the example illustrated in FIG. 10, not two but three 10th gene pairs are found, and it is found that the tenth chromosomes are excessive, that is, an analysis target embryo has chromosome aneuploidy. By using this analysis result and using an embryo from which aneuploidy has not been found among embryos that are being cultured by the embryo culture device 10 to transplant to a patient, it is possible to enhance treatment of infertility treatment, that is, a pregnancy rate. This is because it is known that an embryo having aneuploidy in chromosomes does not implant even when transplanted or a probability of miscarriage at an early pregnancy period is high even if the embryo implants. Moreover, this method can perform non-invasive testing on the embryo 25. Compared to a method for performing testing for aneuploidy of chromosomes by taking out several cells of a trophectderm (TE) from a blastocyst, this method has little influence on an embryo, and can perform aneuploidy analysis in a state where the embryo 25 is immersed in the culture solution CS, so that it is possible to minimize the influence on the embryo.

As described above, according to the dish 191 according to the present embodiment, when testing for aneuploidy is performed using the culture solution CS that has been culturing the embryo 25, even if the culture solution CS is sucked up, the culture solution CS in the microwell 195 containing the embryo 25 reliably remains, so that it is possible to suppress an influence caused by sucking up the culture solution CS. Furthermore, the two rows of the five microwells 195 of the dish 191 according to the present embodiment are disposed close to each other, so that it is easy to move the embryos 25 between the microwells 195, and it is possible to reduce mistakes at a time of transfer. The dish 191 according to the present embodiment makes it possible to image the states of the embryos 25 in the microwells 195 at a time when using the time-lapse embryo culture device. Consequently, it is possible to reduce the number of times of photographing of time-lapse embryo culture without moving the camera module 51 in the state where the plurality of microwells 195 are provided to the one dish 191.

In the present embodiment, the bottom part of the second container part 220 is formed in a round shape toward the center similarly to the first container part 210, so that, when the culture solution CS is sucked up, it is possible to efficiently suck up the culture solution CS in the second container part 220 without leaving the culture solution CS. Moreover, even when all of the culture solution CS in the second container part 220 is sucked up by the pipette 240, the bottom part are bulged by the partition parts 230, so that the liquid level of the culture solution CS in the first container part 210 does not go below this bottom part. Accordingly, the culture solution CS in the microwell 195 in which the embryo 25 exists is not sucked up by mistake.

B. Second Embodiment:

(B-1) Shape of Dish:

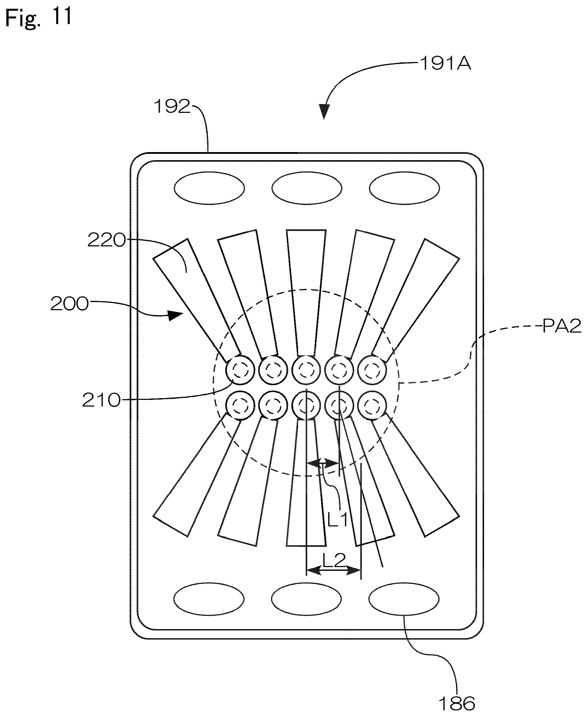

Next, the dish according to the second embodiment will be described. Although the shape of a dish 191A is different as illustrated in FIG. 11 in the second embodiment, configurations other than the shape, that is, the sampling/testing processing routine of the chromosome analysis embryo culture solution are the same as those in the first embodiment. For the dish 191A according to the second embodiment, the container parts 200 having the same shape as that in the first embodiment are used. The number of the container parts 200 and the shapes of the first container parts 210 and the second container parts 220 are also the same. In this regard, in the second embodiment, the first container parts 210 of the 10 container parts 200 are disposed closer to each other than those in the first embodiment. To dispose the first container part 210 close to each other, the container parts 200 are disposed to open more outwardly toward the outer side. Hence, an interval L1 in the row direction of the first container parts 210 in each row is narrower than an interval L2 in the row direction of the second container parts 220 provided in association with the first container parts 210. The interval L2 is a distance between area centers of the second container parts 220. As a result, the dish 191A according to the second embodiment makes it possible to make an imaging range PA2 for imaging the 10 first container parts 210 at a time narrower than an imaging range PA1 according to the first embodiment. Consequently, it is also possible to increase the resolution of the embryo 25 in each microwell 195 imaged by the camera module 51. Note that the size of the imaging range PA2 may be the same size as in the first embodiment, and the number of the container parts 200 may be increased to the six container parts 200 × two rows.

This dish 191A according to the second embodiment makes it possible to narrow the imaging range of each of the plurality of microwells 195, and, moreover, sufficiently secure the sizes of the container parts 200 that store the culture solution CS for testing for aneuploidy of chromosomes. The other function and effects are the same as those in the first embodiment.

(B-2) Modified Example 1:

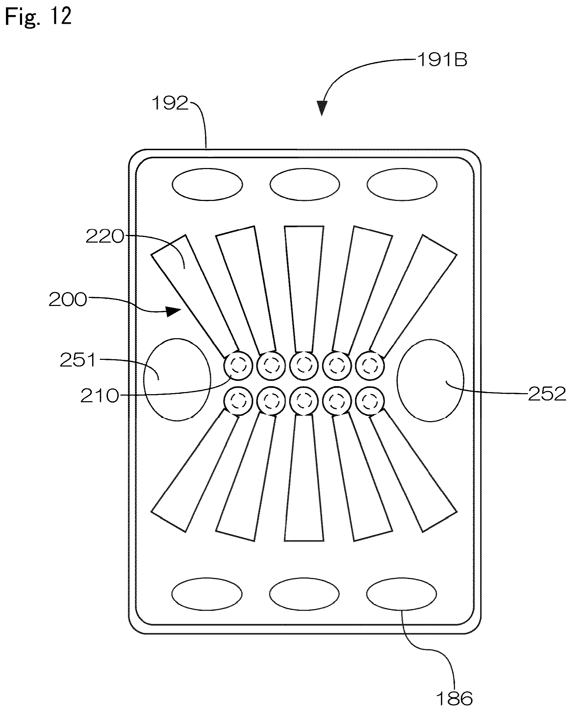

FIG. 12 illustrates a dish 191B according to a modified example of the dish 191A according to the second embodiment. In this example, a replenisher solution container part in which additional injection equilibration drops 251 and 252 of the culture solution CS are disposed are provided in spaces on both sides of an alignment direction of the first container parts 210 formed by disposing the first container parts 210 close to each other. These drops are replenishment culture solutions, and, when the culture solution CS is sucked up from the container part 200, are prepared to additionally inject the culture solution CS into the container part 200 to further continue culturing. During culturing, the culture solution CS is put on the dish 191B in a state where the culture solution CS is covered with the mineral oil OL, and does not evaporate, and has been put in the same environment (such as the temperature and the humidity) as that of the culture solution CS in the container part 200, so that, what is suitable is that, even if the culture solution CS is additionally injected instead of the sucked-up culture solution CS, the added culture solution CS is not different from a culture solution in which the embryo 25 has been immersed.

Although the volume of the container part 200 is 50 $\mu$l, 40 $\mu$l of the culture solution is filled, and 30 $\mu$l of the culture solution is sucked for testing in the above first and second embodiments, the volumes and the quantities described herein are merely examples. When a necessary volume of the culture solution for testing for aneuploidy of chromosomes becomes small, and the volume of the culture solution CS to be sucked becomes, for example, approximately 10 $\mu$l, the volume of the container part 200 may be approximately 20 $\mu$l, and the culture solution CS to be filled may be approximately 15 $\mu$l. Generally, in a case where the volume of the container part 200 is V200, the volume of the culture solution CS to be filled is Vcs, and the volume of the culture solution CS to be sucked for testing is Vout, a relationship of

$$Vout < Vcs \leq V200$$

only needs to be satisfied. Furthermore, what is suitable is that, in a case where the volume of the microwell 195 is Vmw, and

$$Vout + Vmw \leq Vcs$$

holds, when the culture solution CS is sucked, the culture solution remains in the microwell 195.

(B-3) Modified Example 2:

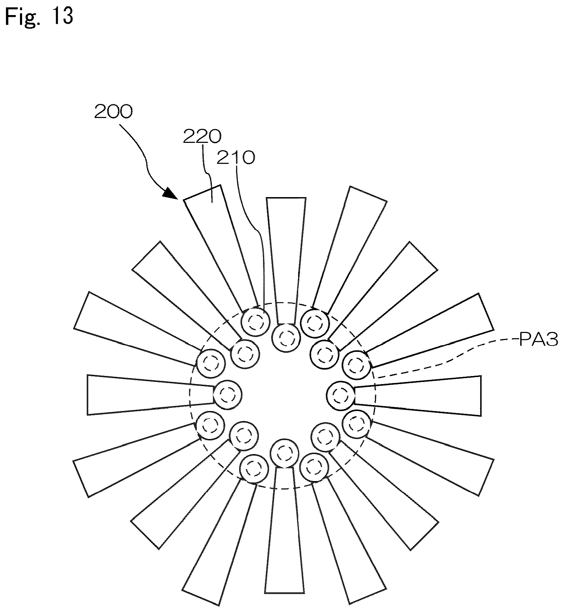

FIG. 13 illustrates another example of arrangement of the container parts 200. In this example, the multiple first container parts 210 of the container parts 200 are disposed much closer to each other. In this example, the container parts 200 are disposed on the circumference, and the eight first container parts 210 each including the microwell 195 on an inner circumferential side, and the eight first container parts 210 on an outer circumferential side, that is, the 16 first container parts 210 in total are provided. Even in this case, it is possible to provide the same function and effect as those in the second embodiment, and it is possible to reduce a necessary imaging range PA3 for imaging the multiple microwells 195.

C. Embryo Culture/Storage/Transplantation Treatment:

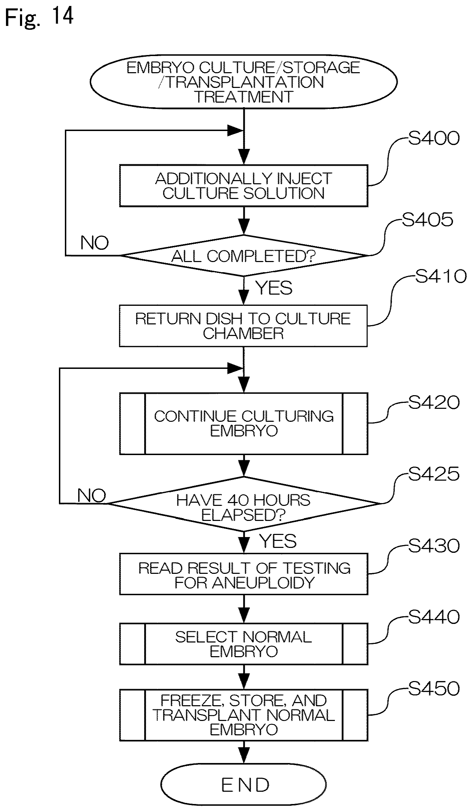

Although how to handle an embryo after the culture solution CS is sucked up is not described in particular in the above-described embodiment, an example of how to handle an embryo for transplantation will be described. FIG. 14 is a flowchart for describing embryo culture/storage/transplantation treatment. After the processing routine illustrated in FIG. 9 is ended, processing of additionally injecting the culture solution CS in each container part 200 is performed (step S400). The processing is performed until additional injection to all of the container parts 200 is completed (step S405).

When additional injection to all of the container parts 200 is completed, processing of returning the dish 191 to the culture chamber R11 of the embryo culture device 10 is performed (step S410). When the lid part 17 of the culture chamber R11 is closed, culturing resumes, and an embryo continues to be cultured (step S420). When culturing resumes, standby is performed until approximately 40 hours have elapsed (step S425), and when 40 hours have elapsed, a result of testing for aneuploidy of chromosomes is read from a result of the next-generation sequencer (step S430). A normal embryo is selected from a plurality of target embryos subjected to testing for aneuploidy of chromosomes by looking at this result (step S440).

When there are embryos from which chromosome aneuploidy has been confirmed among the tested embryos, it is determined that these embryos are not normal embryos, embryos (hereinafter referred to as normal embryos) other than the embryos for which it has been determined that these embryos are not normal are frozen and stored, standby is performed until a patient becomes suitable for transplantation, and the frozen and stored embryos are unfrozen and transplanted to the patient (step S450). As described above, embryo culture/storage/transplantation treatment is ended.

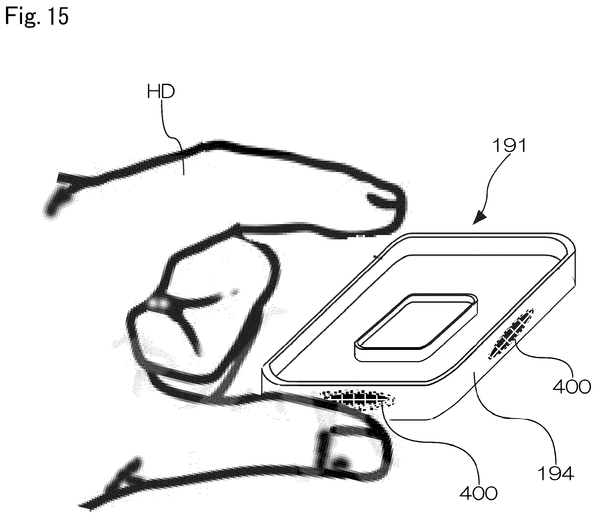

When this dish 191 is taken out from the culture chamber R11 (steps S330 and S350 in FIG. 9) and is returned to the culture chamber R11 (step S410 in FIG. 14), a user directly grips and operates the dish 191 by a hand HD. FIG. 15 illustrates this situation. The dish 191 used in each of the above-described embodiments has a substantially rectangular shape, the user pinches the dish 191 by the thumb and a facing finger such as the middle finger facing the thumb to hold. The outer circumferential wall 194 of the dish 191 is provided with a dent 400 that is a grip assist structure. Consequently, by gripping the dish 191 by the hand HD, the finger fits to the dent 400 of the outer circumferential wall 194, and holds the dish 191 fast. Note that the dent 400 is provided to at least one side of the four sides of the dish 191 having the substantially rectangular shape. The dents 400 may be provided to two portions at which the thumb and the facing finger come into contact. Instead of the dents 400, fine recesses and protrusions may be provided on the outer surface of the outer circumferential wall 194. Although the recesses and the protrusions may be provided only at the places of the dents 400 in this case, the outer side of the outer circumferential wall 194 may be a wide uneven surface. The recesses and the protrusions may be formed by making the surface of a mold uneven at a time of formation of the dish 191, or may be formed by roughening the surface by a method such as sandblasting later. Note that illustration of the recess portion 197 is omitted in FIG. 15.

D. Other Embodiment:

(1) An embryo culture dish according to the present disclosure can be implemented as an embryo culture dish that includes a plurality of wells that each contain an embryo together with a culture solution. In this embryo culture dish, the plurality of wells may be disposed independently and adjacently at a bottom part of the embryo culture dish, a recess part that can retain the culture solution together with the well may be formed per well at the bottom part independently from another recess part, a movement block part that permits circulation of the culture solution and blocks movement of the embryo may be provided between the well and the recess part, and a volume of the recess part may be equal to or more than a volume of a culture solution to be sucked up for testing for aneuploidy of a chromosome of the embryo cultured in the well.

According to such an embryo culture dish, the plurality of wells are adjacent, so that, if this embryo culture dish is used for an embryo culture device or the like, distances between the plurality of wells are short and are easy to handle, and it is possible to perform processing of sucking up the culture solution for testing for aneuploidy of chromosomes of an embryo while suppressing an influence on the embryo that is being cultured. Moreover, the movement block part is provided between the wells and the recess parts, so that it is possible to suck up the culture solution around the embryo, too, while leaving in the well the embryo to be cultured in the well. Furthermore, the distances between the plurality of wells are short, so that it is easy to move an embryo in a well, and it is possible to reduce mistakes at a time of transfer. Furthermore, by using the embryo culture dish for a time-lapse embryo culture device, it is possible to image a plurality of wells at a time. Consequently, it is easy to perform non-invasive testing for aneuploidy of chromosomes on embryos, and it is possible to expect that an embryo transfer success rate (a pregnancy rate of a patient) in consideration of results of testing for aneuploidy increases.

The number of wells per embryo culture dish may be plural, and may be any number as long as the number of wells is two at minimum and a container part can be installed by disposing the wells adjacently. The wells may have a hemispherical shape or may have an oval shape in plan view in accordance with the shape of an embryo to be contained, and a cross section in a vertical direction may be a parabolic shape. Furthermore, the plurality of wells do not necessarily need to have the same shape, and may individually have different shapes. By so doing, it is also possible to visually recognize which embryo in which well in a visual field of a microscope is to be operated.

(2) According to this configuration, at least part of the plurality of wells may be aligned in two rows and disposed, and each of the recess parts provided in association with the wells disposed in the one row may be provided on an opposite side to the recess part provided in association with the well of the other row with the well interposed therebetween, and have a shape having a predetermined length from the corresponding well to an outer side. By so doing, it is easy to dispose a plurality of wells adjacently and dispose a container part having a sufficient size. A predetermined length may be adjusted according to the size of the container part and a liquid amount of the culture solution to be contained in particular. The container part may have a rectangular shape, or may have a trapezoidal shape whose length in the horizontal direction becomes longer apart from the well. Naturally, the container part may have an elliptical shape in plan view or an oval shape. Furthermore, the container part may entirely have the uniform depth in the depth direction, or may have a curved shape whose depth becomes deeper toward a center part similarly to the well. In a case of the latter, when the culture solution is sucked up, the culture solution gathers at the deepest part, so that it is possible to efficiently suck up the culture solution in the container part.

(3) According to this configuration, an interval in a row direction of the wells in each of the rows may be narrower than an interval in the row direction of the recess parts provided in association with the wells. By so doing, it is possible to more efficiently dispose the wells, and increase the number of wells to be provided to the embryo culture dish. Consequently, by using this embryo culture dish for the time-lapse embryo culture device or the like, it is also possible to increase the number of wells that can be imaged at a time.

(4) According to this configuration, all of the wells in the embryo culture dish may be disposed in a range in which a camera provided to a time-lapse embryo culture device that contains the embryo culture dish can image the embryo culture dish without relatively moving with respect to the embryo culture dish. By so doing, the camera provided to the time-lapse embryo culture device can image all wells at a time, and it is not necessary to move the camera with respect to the embryo culture dish. Naturally, one embryo culture dish may be provided with a plurality of sets including pluralities of wells and recess parts, and may be imaged sequentially by moving the camera.

(5) According to this configuration, the movement block part may include a passage portion that connects the wells and the recess parts and is shallower than depths of the wells. By so doing, even when all of the culture solution in the recess part is sucked up by a pipette or the like, the depth of the movement block part is shallower than the depth of the well, so that a liquid level of the culture solution in the well does not go below this passage portion. Accordingly, the culture solution in the well in which the embryo to be cultured exists is not sucked up by mistake. Naturally, the movement block part only needs to be able to block movement of the embryo from the well, and the depth of the movement block part may be substantially the same as the depth of the well. Although it is also possible to suck up all of the culture solution in the well in this case, if the culture solution around the embryo needs to be left, a suck-up amount may be adjusted. For example, a distal end of the pipette used to suck up the culture solution may be provided with an adapter that separates the pipette a predetermined distance from the bottom part of the recess part. By so doing, it is possible to leave the culture solution of a predetermined height from the bottom part of the recess part by sucking up the culture solution using the pipette.

(6) According to this configuration, the movement block part may include a passage portion that is provided at a connection portion between the wells and the recess parts and includes a gap through which the culture solution circulates, and the gap of the passage portion may be narrower than a minimum diameter of the embryo contained in the well. By so doing, it is possible to easily block movement of the embryo in the well. The passage may be simply formed as a gap narrower than the size of the embryo. In this case, the width of the passage itself may be narrowed or a fence or a mesh may be provided to a passage having a wide width to block movement of the embryo.

(7) According to this configuration, the well may include at a bottom part of the well a microwell that is smaller than an outer shape of the well and is larger than an outer shape of the contained embryo. By so doing, it is possible to bring the position of the embryo into the microwell, and limit the position of the embryo to be imaged. Furthermore, it is possible to restrict movement of the embryo that is being cultured, which is desirable for culturing the embryo. Naturally, the microwell may not be provided. A structure that the bottom part of the well is provided with a bulged part having a slightly smaller diameter than the diameter of the embryo and having a ring shape also makes it possible to restrict movement of the embryo in the well.

(8) This configuration may further include on a bottom surface of the embryo culture dish a replenisher solution container part that contains a replenishment culture solution for replenishing the culture solution to the recess part. By so doing, it is possible to easily replenish an equilibration culture solution accustomed to an environment in which culturing is being performed.

(9) This configuration may include an outer circumferential wall that partitions an outer circumference, and an outer surface of the outer circumferential wall may have a grip assist structure that assists grip of a hand of a user when the user grips the embryo culture dish. By so doing, it is possible to easily handle the embryo culture dish.

(10) This configuration may include: filling the culture solution in the wells and the recess parts in the embryo culture dish; containing an embryo to culture in the well filled with the culture solution; covering the wells and the recess parts with an oil, the oil suppressing evaporation of the culture solution, and the wells and the recess parts being filled with the culture solution; sucking up a predetermined volume of the culture solution from the recess part a predetermined time after the embryo is contained in the well; and providing the sucked-up culture solution for analysis of an analyzer that can perform chromosome analysis for aneuploidy using a DNA included in the culture solution. By so doing, it is possible to implement non-invasive testing for aneuploidy of chromosomes for embryos, and there is little influence on testing target embryos. Consequently, it is also possible to expect that an embryo transfer success rate (a pregnancy rate of a patient) in consideration of results of testing for aneuploidy increases.

The present disclosure is not limited to the above-described embodiments, and can be implemented by various configurations without departing from the gist of the present disclosure. For example, technical features in the embodiments corresponding to technical features in each aspect described in the summary can be replaced or combined as appropriate to solve part or all of the above-described problems or achieve part or all of the above-described effects. Furthermore, the technical features that are not described as indispensable in this description can be deleted as appropriate.

The present disclosure is not limited to the above-described embodiments, and can be implemented by various configurations without departing from the gist of the present disclosure. For example, technical features in the embodiments corresponding to technical features in the aspects described in the summary can be replaced or combined as appropriate to solve part or all of the above-described problems or achieve part or all of the above-described effects. Furthermore, the technical features that are not described as indispensable in this description can be deleted as appropriate. For example, part of the components that are implemented by hardware in the above embodiments can be implemented by software.

REFERENCE SIGNS LIST

10 ... EMBRYO CULTURE DEVICE, 11 TO 24 ... CULTURE UNIT, 17 ... LID PART, 18 ... GAS PORT, 20 ... CASE MAIN BODY, 25 ... EMBRYO, 26 ... GENERAL-PURPOSE CONNECTOR, 27 ... FILTER, 28 ... FILTER PORT, 43 ... SUPPLY PORT, 44 ... DISCHARGE PORT, 51 ... CAMERA MODULE, 52 ... LENS MODULE, 60... CONTROL UNIT, 61 ... CPU, 62 ... ROM, 63 ... RAM, 64 ... MEMORY INTERFACE, 65 ... MEMORY CARD, 66 ... GENERAL-PURPOSE I/O INTERFACE, 67 ... CULTURE CHAMBER INTERFACE, 68 ... CAMERA INTERFACE, 70 ... DISPLAY, 71 ... DRIVING DEVICE, 72 ... WARNING DEVICE, 73 ... MIXTURE ADJUSTMENT DEVICE, 84 ... MIXTURE SUPPLY PIPE, 91 ... DISPLAY, 95 ... CONTROL DEVICE, 96 ... DIAL, 97 ... BASE PART, 170 ... ILLUMINATION UNIT, 180 ... HOLDING FRAME, 182 ... PROTRUSION PART, 186 ... DROP ARRANGEMENT PART, 191, 191A, 191B ... DISH, 192 ... WALL, 194 ... OUTER CIRCUMFERENTIAL WALL, 195 ... MICROWELL, 196 ... BOTTOM PART, 197 ... RECESS PORTION, 198 ... OPENING, 200 ... CONTAINER PART, 210 ... FIRST CONTAINER PART, 220 ... SECOND CONTAINER PART, 230 ... PARTITION PART, 240 ... PIPETTE, 251 ... ADDITIONAL INJECTION EQUILIBRATION DROP, 300 ... NEXT-GENERATION SEQUENCER, 310 ... CONTAINER PART, 320 ... OPERATION UNIT, 330 ... DISPLAY UNIT, HD ... HAND

**Claims**

1. An embryo culture dish that comprises a plurality of wells that each contain an embryo together with a culture solution, wherein

   the plurality of wells are disposed independently and adjacently at a bottom part of the embryo culture dish,
   a recess part that can retain the culture solution together with the well is formed per well at the bottom part independently from another recess part,
   a movement block part that permits circulation of the culture solution and blocks movement of the embryo is provided between the well and the recess part, and
   a volume of the recess part is equal to or more than a volume of a culture solution to be sucked up for testing for aneuploidy of a chromosome of the embryo cultured in the well.

2. The embryo culture dish according to claim 1, wherein at least part of the plurality of wells are aligned in two rows and disposed, and each of the recess parts provided in association with the wells disposed in the one row is provided on an opposite side to the recess part provided in association with the well of the other row with the well interposed therebetween, and has a shape having a predetermined length from the corresponding well to an outer side.

3. The embryo culture dish according to claim 2, wherein an interval in a row direction of the wells in each of the rows is narrower than an interval in the row direction of the recess parts provided in association with the wells.

4. The embryo culture dish according to any one of claims 1 to 3, wherein all of the wells in the embryo culture dish are disposed in a range in which a camera provided to a time-lapse embryo culture device that contains the embryo culture dish can image the embryo culture dish without relatively moving with respect to the embryo culture dish.

5. The embryo culture dish according to any one of claims 1 to 4, wherein the movement block part includes a passage portion that connects the wells and the recess parts and is shallower than depths of the wells.

6. The embryo culture dish according to any one of claims 1 to 5, wherein

the movement block part includes a passage portion that is provided at a connection portion between the wells and the recess parts and includes a gap through which the culture solution circulates, and
the gap of the passage portion is narrower than a minimum diameter of the embryo contained in the well.

7. The embryo culture dish according to any one of claims 1 to 6, wherein the well includes at a bottom part of the well a microwell that is smaller than an outer shape of the well and is larger than an outer shape of the contained embryo.

8. The embryo culture dish according to any one of claims 1 to 7, further comprising on a bottom surface of the embryo culture dish a replenisher solution container part that contains a replenishment culture solution for replenishing the culture solution to the recess part.

9. The embryo culture dish according to any one of claims 1 to 8, further comprising an outer circumferential wall that partitions an outer circumference, wherein an outer surface of the outer circumferential wall has a grip assist structure that assists grip of a hand of a user when the user grips the embryo culture dish.

10. A method for sampling a chromosome analysis embryo culture solution comprising:

filling the culture solution in the wells and the recess parts in the embryo culture dish according to any one of claims 1 to 9;
containing an embryo to culture in the well filled with the culture solution;
covering the wells and the recess parts with an oil, the oil suppressing evaporation of the culture solution, and the wells and the recess parts being filled with the culture solution;
sucking up a predetermined volume of the culture solution from the recess part a predetermined time after the embryo is contained in the well; and
providing the sucked-up culture solution for analysis of an analyzer that can perform chromosome analysis for aneuploidy using a DNAincluded in the culture solution.

Fig. 1

Fig. 2

Fig. 3

Fig. 4

Fig.5

Fig. 6

Fig. 7

Fig. 8

Fig. 9

```
        ┌──────────────────────────────┐
        │  SAMPLE/INSPECTION PROCESSING │
        │     OF CHROMOSOME ANALYSIS    │
        │    EMBRYO CULTURE SOLUTION    │
        └──────────────────────────────┘
                      │
                      ▼
        ┌──────────────────────────────┐        S300
        │    FILL 40 μl OF CULTURE      │
        │ SOLUTION IN ALL CONTAINER     │
        │           PARTS              │
        └──────────────────────────────┘
                      │
                      ▼
        ┌──────────────────────────────┐        S310
        │        COVER WITH OIL         │
        └──────────────────────────────┘
                      │
                      ▼
        ┌──────────────────────────────┐        S320
        │  CONTAIN TREATED EGG IN       │
        │         MICROWELL             │
        └──────────────────────────────┘
                      │
                      ▼
    NO      ◇ ALL COMPLETED? ◇                   S325
                      │ YES
                      ▼
        ┌──────────────────────────────┐        S330
        │           SET DISH            │
        └──────────────────────────────┘
                      │
                      ▼
        ┌──────────────────────────────┐        S340
        │   TIME-LAPSE CULTURE          │
        │        PROCESSING             │
        └──────────────────────────────┘
                      │
                      ▼
    NO      ◇ HAVE 72 HOURS ELAPSED? ◇           S345
                      │ YES
                      ▼
        ┌──────────────────────────────┐        S350
        │        TAKE OUT DISH          │
        └──────────────────────────────┘
                      │
                      ▼
        ┌──────────────────────────────┐        S360
        │     PROCESSING OF             │
        │  SUCKING UP 30 μl OF          │
        │    CULTURE SOLUTION           │
        └──────────────────────────────┘
                      │
                      ▼
    NO      ◇ ALL COMPLETED? ◇                   S365
                      │ YES
                      ▼
        ┌──────────────────────────────┐        S370
        │  TESTING FOR ANEUPLOIDY       │
        │   WITH GENE SEQUENCER         │
        └──────────────────────────────┘
                      │
                      ▼
                 ( END )
```

Fig. 10

Fig. 11

Fig. 12

Fig. 13

Fig. 14

```
        ( EMBRYO CULTURE/STORAGE
          /TRANSPLANTATION
              TREATMENT )
                  │
                  ▼
        ┌──────────────────────┐
        │ ADDITIONALLY INJECT  │──── S400
        │   CULTURE SOLUTION   │
        └──────────────────────┘
                  │
                  ▼
  NO        < ALL COMPLETED? >──── S405
                  │ YES
                  ▼
        ┌──────────────────────┐
        │ RETURN DISH TO CULTURE│──── S410
        │       CHAMBER        │
        └──────────────────────┘
                  │
                  ▼
        ┌──────────────────────┐
        │ CONTINUE CULTURING   │──── S420
        │       EMBRYO         │
        └──────────────────────┘
                  │
                  ▼
  NO      < HAVE 40 HOURS >──── S425
              ELAPSED?
                  │ YES
                  ▼
        ┌──────────────────────┐
        │ READ RESULT OF TESTING│──── S430
        │   FOR ANEUPLOIDY     │
        └──────────────────────┘
                  │
                  ▼
        ┌──────────────────────┐
        │ SELECT NORMAL        │──── S440
        │       EMBRYO         │
        └──────────────────────┘
                  │
                  ▼
        ┌──────────────────────┐
        │ FREEZE, STORE, AND   │──── S450
        │ TRANSPLANT NORMAL    │
        │       EMBRYO         │
        └──────────────────────┘
                  │
                  ▼
              ( END )
```

Fig. 15

# EP 4 671 355 A1

## INTERNATIONAL SEARCH REPORT

| | International application No. |
|---|---|
| | **PCT/JP2023/006407** |

| | |
|---|---|
| **A.**    **CLASSIFICATION OF SUBJECT MATTER** | |

*C12M 3/00*(2006.01)i; *C12N 5/073*(2010.01)i; *C12M 1/34*(2006.01)i
FI:   C12M3/00 Z; C12M1/34 B; C12N5/073

According to International Patent Classification (IPC) or to both national classification and IPC

| **B.**    **FIELDS SEARCHED** |
|---|

Minimum documentation searched (classification system followed by classification symbols)

     C12M1/00-3/10; C12N5/00-5/28

Documentation searched other than minimum documentation to the extent that such documents are included in the fields searched

     Published examined utility model applications of Japan 1922-1996
     Published unexamined utility model applications of Japan 1971-2023
     Registered utility model specifications of Japan 1996-2023
     Published registered utility model applications of Japan 1994-2023

Electronic data base consulted during the international search (name of data base and, where practicable, search terms used)

     JSTPlus/JMEDPlus/JST7580 (JDreamIII); CAplus/MEDLINE/EMBASE/BIOSIS (STN); PubMed

| **C.**    **DOCUMENTS CONSIDERED TO BE RELEVANT** |
|---|

| Category* | Citation of document, with indication, where appropriate, of the relevant passages | Relevant to claim No. |
|---|---|---|
| Y | JP 2017-063744 A (DAI NIPPON PRINTING CO., LTD.) 06 April 2017 (2017-04-06) <br>      claims, paragraphs [0023]-[0025], [0053]-[0055], [0063]-[0065], [0097]-[0100], fig. 4-5 | 1-10 |
| Y | JP 2020-532999 A (COOPERGENOMICS INC.) 19 November 2020 (2020-11-19) <br>      claims, paragraph [0128] | 1-10 |
| Y | JP 2018-061453 A (ANIMAL STEM CELL CO., LTD.) 19 April 2018 (2018-04-19) <br>      claims | 1-10 |
| Y | JP 2022-152184 A (ASADA LADIES CLINIC) 12 October 2022 (2022-10-12) <br>      claims, fig. 4 | 1-10 |
| Y | TIME-LAPSE DISHES. Vitrolife [online]. 20 January 2022, Internet:<URL: https:// web.archive.org/web/20220120015003/https://www.vitrolife.com/products/time-lapse-systems/time-lapse-dishes/>, [retrieved on 05 February 2023] <br>      in particular, "EmbryoSlide+ ic 8 culture dish", "EmbryoScope+ holds 15 patient dishes with up to 16 embryos each" | 1-10 |

☑ Further documents are listed in the continuation of Box C.     ☑ See patent family annex.

| *   Special categories of cited documents: | "T"   later document published after the international filing date or priority date and not in conflict with the application but cited to understand the principle or theory underlying the invention |
|---|---|
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **02 May 2023** | **16 May 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| International application No. |
| --- |
| **PCT/JP2023/006407** |

### C. DOCUMENTS CONSIDERED TO BE RELEVANT

| Category* | Citation of document, with indication, where appropriate, of the relevant passages | Relevant to claim No. |
| --- | --- | --- |
| A | 辻　暖永　ほか, ヒトzona-free胚に適した培養法の探索－dish形状に着目した検討から, 日本卵子学会誌, 2017, vol. 2, pp. S66:O-114, (TSUJI, Haruhisa et al. Journal of Ova Research.), non-official translation (Search for culture method suitable for human zona-free embryos -From study focusing on shape of dish)<br>　　entire text | 1-10 |

Form PCT/ISA/210 (second sheet) (January 2015)

**INTERNATIONAL SEARCH REPORT**
Information on patent family members

International application No.

**PCT/JP2023/006407**

| Patent document cited in search report | Publication date (day/month/year) | Patent family member(s) | Publication date (day/month/year) |
|---|---|---|---|
| JP 2017-063744 A | 06 April 2017 | (Family: none) | |
| JP 2020-532999 A | 19 November 2020 | WO 2019/051244 A1 claims, paragraph [0128] | |
| JP 2018-061453 A | 19 April 2018 | (Family: none) | |
| JP 2022-152184 A | 12 October 2022 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014507664 W